# EUROPEAN PATENT SPECIFICATION

(11) **EP 1 375 518 B1**
(45) Date of publication and mention of the grant of the patent: **22.10.2008**
(21) Application number: 02713293.5
(22) Date of filing: 04.04.2002
(51) Int. Cl.: C07K 16/18, C07K 7/06, C07K 7/08, C12N 15/09, C12P 21/08, A61K 39/395, A61K 45/00, A61P 37/02, A61P 29/00, A61P 19/02, A61P 43/00

(54) **ANTI-OSTEOPONTIN ANTIBODY AND USE THEREOF**
ANTI-OSTEOPONTIN-ANTIKÖRPER UND DESSEN VERWENDUNG
ANTICORPS DIRIG CONTRE L'OST OPONTINE, ET SON UTILISATION

(30) Priority: 05.04.2001 JP 2001107578; 25.09.2001 JP 2001290700
(43) Date of publication of application: 02.01.2004
(60) Divisional application: 06120039.0
(73) Proprietor: Immuno-Biological Laboratories Co., Ltd., Takasaki-shi, Gunma 370-0831 (JP); Astellas Pharma Inc., Tokyo 103-8411 (JP)
(72) Inventor: UEDE, Toshimitsu, Sapporo-shi, Hokkaido 004-0835 (JP); KON, Shigeyuki, Sapporo-Shi, Hokkaido 001-0017 (JP); SAEKI, Yukihiko, Izumi-shi, Osaka 594-1118 (JP); YOKOSAKI, Yasuyuki, Hiroshima-shi, Hiroshima 731-5135 (JP); NODA, Masaki, Shibuya-ku, Tokyo 150-0022 (JP); YAMAMOTO, Nobuchika c/o Astellas Pharma Inc., Chuo-ku, Tokyo 103-8477 (JP)
(74) Representative: Gille Hrabal Struck Neidlein Prop Roos
(86) International application number: PCT/JP2002/003382
(87) International publication number: WO 2002/081522

(56) References cited:
- EP-A2- 0 705 842
- WO-A-00/63247
- WO-A-01/71358
- BAUTISTA D S ET AL: "A monoclonal antibody against osteopontin inhibits RGD-mediated cell adhesion to osteopontin." ANNALS OF THE NEW YORK ACADEMY OF SCIENCES. 21 APR 1995, vol. 760, 21 April 1995 (1995-04-21), pages 309-311, XP009033073 ISSN: 0077-8923
- HOTTA H ET AL: "Detection of various epitopes of murine osteopontin by monoclonal antibodies." BIOCHEMICAL AND BIOPHYSICAL RESEARCH COMMUNICATIONS. 2 APR 1999, vol. 257, no. 1, 2 April 1999 (1999-04-02), pages 6-11, XP002286858 ISSN: 0006-291X
- RITTLING S R ET AL: "Detection of mouse osteopontin by western blotting." BIOCHEMICAL AND BIOPHYSICAL RESEARCH COMMUNICATIONS. 18 SEP 1998, vol. 250, no. 2, 18 September 1998 (1998-09-18), pages 287-292, XP002286859 ISSN: 0006-291X
- KON S ET AL: "Mapping of functional epitopes of osteopontin by monoclonal antibodies raised against defined internal sequences" JOURNAL OF CELLULAR BIOCHEMISTRY, WILEY-LISS INC, US, vol. 84, no. 2, 15 November 2001 (2001-11-15), pages 420-432, XP002961001 ISSN: 0730-2312
- SAKATA M ET AL: "Autoantibodies to osteopontin in patients with osteoarthritis and rheumatoid arthritis." THE JOURNAL OF RHEUMATOLOGY. JUL 2001, vol. 28, no. 7, July 2001 (2001-07), pages 1492-1495, XP009033062 ISSN: 0315-162X
- BAUTISTA DIOSDADO S. ET AL.: 'Inhibition of Arg-Gly-Asp (RGD)-mediated cell adhesion to osteopontin by a monoclonal antibody against osteopontin' THE JOURNAL OF BIOLOGICAL CHEMISTRY vol. 269, no. 37, 1994, pages 23280 - 23285, XP002953832
- BARRY SIMON T. ET AL.: 'Analysis of the alpha4beta1 integrin-osteopontin interaction' EXPERIMENTAL CELL RESEARCH vol. 258, 2000, pages 342 - 351, XP001020897
- YASUYUKI YOKOSAKI ET AL.: 'The integrin alpha9beta1 binds to a novel recognition sequence (SVVYGLR) in the trombin-cleaved amino-terminal fragment of osteopontin' THE JOURNAL OF BIOLOGICAL CHEMISTRY vol. 274, no. 51, 1994, pages 36328 - 36334, XP002953833
- KON S. ET AL: '"ANTIBODIES TO DIFFERENT PEPTIDES IN OSTEOPONTIN REVEAL COMPLEXITIES IN THE VARIOUS SECRETED FORMS"' JOURNAL OF CELLULAR BIOCHEMISTRY vol. 77, no. 3, April 2000, pages 487 - 498
- ATTUR M.G. ET AL: '"OSTEOPONTIN AN INTRINSIC INHIBITOR OF INFLAMMATION IN CARTILAGE",' ARTHRITIS AND RHEUMATISM vol. 44, no. 3, March 2001, pages 578 - 584
- PETROW P.K. ET AL: '"EXPRESSION OF OSTEOPONTIN MESSENGER RNA AND PROTEIN IN RHEUMATOID ARTHRITIS EFFECTS OF OSTEOPONTIN ON THE RELEASE OF COLLAGENASE 1 FROM ARTICULAR CHONDROCYTES AND SYNOVIAL FIBROBLASTS"' ARTHRITIS AND RHEUMATISM vol. 43, no. 7, July 2000, pages 1597 - 1605

<110> Immuno-Biological Laboratories Co., Ltd. Fujisawa Pharmaceutical Co., Ltd.
<120> Anti-osteopontin antibody and use thereof
<130> PF-020002-WO
<140>
   <141>
<150> JP 2001-290700
   <151> 2001-09-25
<150> JP 2001-107578
   <151> 2001-04-05
<160> 18
<170> PatentIn Ver. 2.1
<210> 1
   <211> 18
   <212> PRT
   <213> Artificial Sequence
<220>
   <223> Description of Artificial Sequence: (1) hOPN5
<400> 1
   Cys Val Asp Thr Tyr Asp Gly Arg Gly Asp Ser Val Val Tyr Gly Leu
    1               5                  10                  15
   Arg Ser
<210> 2
   <211> 19
   <212> PRT
   <213> Artificial Sequence
<220>
   <223> Description of Artificial Sequence: (2) hOPN3
<400> 2
   Lys Ser Lys Lys Phe Arg Arg Pro Asp Ile Gln Tyr Pro Asp Ala Thr
   1 5 10 15
   Asp Glu Cys
<210> 3
   <211> 16
   <212> PRT
   <213> Artificial Sequence
<220>
   <223> Description of Artificial Sequence: (3) hOPN1
<400> 3
   Ile Pro Val Lys Gln Ala Asp Ser Gly Ser Ser Glu Glu Lys Gln Cys
   1 5 10 15
<210> 4
   <211> 35
   <212> DNA
   <213> Artificial Sequence
<220>
   <223> Description of Artificial Sequence:OPN-5
<400> 4
   cgggatccac taccatgaga attgcagtga tttgc 35
<210> 5
   <211> 36
   <212> DNA
   <213> Artificial Sequence
<220>
   <223> Description of Artificial Sequence:OPN-3
<400> 5
   ccgctcgagt taattgacct cagaagatgc actatc 36
<210> 6
   <211> 37
   <212> DNA
   <213> Artificial Sequence
<220> .
   <223> Description of Artificial Sequence:OPNct-3
<400> 6
   acacagcatt cttttccaca gaacttccag aatcagc 37
<210> 7
   <211> 35
   <212> DNA
   <213> Artificial Sequence
<220>
   <223> Description of Artificial Sequence:OPNet-5
<400> 7
   tgaggaaaag aatgctgtgt cctctgaaga aaacc 35
<210> 8
   <211> 32
   <212> DNA
   <213> Artificial Sequence
<220>
   <223> Description of Artificial Sequence:OPNnh-3
<400> 8
   gcctcgagtt acctcagtcc ataaaccaca ct 32
<210> 9
   <211> 31
   <212> DNA
   <213> Artificial Sequence
<220>
   <223> Description of Artificial Sequence :OPNch-3
<400> 9
   tcttagattt ggcacaggtg atgcctagga g 31
<210> 10
   <211> 31
   <212> DNA
   <213> Artificial Sequence
<220> -
   <223> Description of Artificial Sequence:OPNch-5
<400> 10
   cacctgtgcc aaatctaaga agtttcgcag a 31
<210> 11
   <211> 18
   <212> PRT
   <213> Artificial Sequence
<220>
   <223> Description of Artificial Sequence:peptidel
<400> 11
   Cys Val Asp Thr Tyr Asp Gly Arg Gly Asp Ser Val Val Tyr Gly Leu
   1 5 10 15
   Arg Ser
<210> 12
   <211> 17
   <212> PRT
   <213> Artificial Sequence
<220>
   <223> Description of Artificial Sequence:peptide2
<400> 12
   Cys Ile Asp Ser Gln Glu Leu Ser Lys Val Ser Arg Glu Phe His Ser
   1 5 10 15
   His
<210> 13
   <211> 17
   <212> PRT
   <213> Artificial Sequence
<220>
   <223> Description of Artificial Sequence:M5peptide
<400> 13
   Cys Val Asp Val Pro Asn Gly Arg Gly Asp Ser Leu Ala Tyr Gly Leu
   1 5 10 15
   Arg
<210> 14
   <211> 7
   <212> PRT
   <213> Artificial Sequence
<220>
   <223> Description of Artificial Sequence:fragment peptide
<400>, 14
<210> 15
   <211> 11
   <212> PRT
   <213> Artificial Sequence
<220>
   <223> Description of Artificial Sequence fragment peptide
<400> 15
   Arg Gly Asp Ser Val Val Tyr Gly Leu Arg Ser
   1 5 10
<210> 16
   <211> 17
   <212> PRT
   <213> Artificial Sequence
<220>
   <223> Description of Artificial Sequence:fragment peptide
<400> 16
   Val Asp Thr Tyr Asp Gly Arg Gly Asp Ser Val Val Tyr Gly Leu Arg
   1 5 10 15
   Ser
<210> 17
   <211> 11
   <212> PRT
   <213> Artificial Sequence
<220>
   <223> Description of Artificial Sequence:fragment peptide
<400> 17
   Arg Gly Asp Ser Leu Ala Tyr Gly Leu Arg Ser
   1 5 10
<210> 18
   <211> 17
   <212> PRT
   <213> Artificial Sequence
<220>
   <223> Description of Artificial Sequence:fragment peptide
<400> 18
   Val Asp Thr Tyr Asp Gly Arg Gly Asp Ser Leu Ala Tyr Gly Leu Arg
   1 5 10 15
   Ser

## Description

### Technical Field of the invention

The present invention relates to an anti-osteopontin antibody produced by the hybridoma deposited as FERM BP-7883 and a therapeutic agent for treating autoimmune diseases, rheumatism and rheumatoid arthritis, comprising the antibody, as well as a use of the antibody for the manufacture of a medicament for treating said diseases.

### Background Art

Osteopontin (referred to as "OPN" hereinbelow) is an acidic, calcium-binding glycoprotein abundant in bone. It has been known that three types of human OPN isoforms namely osteopontin-a (referred to as "OPN-a" hereinbelow), osteopontin-b (referred to as "OPN-b" hereinbelow) and osteopontin-c (referred to as "OPN-c" hereinbelow), are naturally generated by alternative splicing (Y. Saitoh et al., (1995): Laboratory Investigation, 72, 55-63). It has beenbelieved that among them, the precursor of OPN-a has an amino acid sequence shown as SQ ID NO.1 below in the Sequence Listing, where the signal peptide is cleaved on secretion, so that the mature formOPN-a of I17-N314 isprepared. Additionally, the mature OPN is cleaved at the C-terminal side of the 168-th residue arginine with thrombin from a biological organism into two fragments N-terminal and C-terminal.

OPN described above has various physiologically, pathologically significant functions, for example cell adhesion, cell migration, tumorigenesis, immune response and inhibition of complement-mediated cytolysis. Various types of receptors on cellular surface mediate the various functions. OPN has, the RGD sequence therein (for example, OPN-a has the sequence from the residue at position 159 to the residue at position 161). Integrin species recognizing the RGD sequence such as αVβ3, αVβ1 and αVβ5 are major OPN receptors; specifically, the integrin species αVβ3, αVβ1 and αVβ5 mediate cell adhesion in vascular smooth muscle cells. Further, αVβ3 is involved in the migrations of macrophages, lymphocytes, endothelial cells, and smooth muscle cells and the like.

Further, research works so far have elucidated that OPN also binds through the sequence SVVYGLR to α9β1, α4β1 and α4β7 integrin species and that a difference in the mode is also found such that α4β1 binds to both OPN not yet cleaved with thrombin (non-cleavage-type OPN) and the N-terminal fragment of thrombin-cleaved OPN (cleavage-type OPN), while α9β1 binds only to the thrombin-cleavage-type OPN. (Y. Yokosaki et al., (1999) : The Journal of Biological Chemistry 274, 36328-36334/P. M. Green et al., (2001): FEBS Letters 503, 75-79/S. T. Barry et al., (2000): Experimental Cell Research 258, 342-351). These integrin subunits α9 and α4 or the integrin subunits β1 and β7 are highly similar in terms of amino acid sequence to each other. Additionally, the integrin species α4β1 and α4β7 are mainly found in lymphocytes and monocytes, while in neutrophils, the integrin species are expressed very slightly. Alternatively, α9β1 is highly expressed selectively in neutrophils and has functions essential for neutrophil migration through VCAM-1 and Tenascin-C. Additionally, the integrin is also expressed diversely in muscular cells, epithelial cells and liver cells. As described above, the cytoplasm domains of the integrin subunits α4 and α9 cooperatively promote leukocyte migration toward inflammatory sites and aggregation therein, via individual cellular signal transmission pathways subtly differing from each other, to enhance their infiltration activities. In such manner, the integrin subunits are involved in various inflammatory reactions.

As described above, various types of integrin species promote leukocyte migration and are thus involved in inflammatory reactions. Therefore, pharmaceutical substances inhibiting these integrin activities may potentially have a possibility as an anti-inflammatory agent. For example, the integrin αVβ3 is expressed in osteoclast cells, vascular endothelial cells and smooth muscle cells and the like. An anti-αVβ3 antibody is now under way of development, which will work to inhibit the binding between the integrin αVβ3 and various binding ligands thereof to potentially exert for example an action to suppress articular damages.

Because receptors of the integrin family commonly emerge in diverse tissues to provide essential functions for the control of vital activities, however, the use of antibodies against integrin for the therapeutic treatment of rheumatoid arthritis and osteoarthritis may possibly elicit the same inhibition at other sites and may also cause the occurrence of side effects.

Additionally, WO 01/71358 discloses a method for screening for a substance inhibiting the binding between the α4 integrin and osteopontin and a method for therapeutically treating inflammatory diseases, using the substance recovered by such screening.

WO01/71358 discloses a method of screening inhibitors of osteopontin. It is a document according to Article 54(3) EPC. It describes antibodies recognizing the SVVYGLR motif or RGDSVVYGLR motif of osteopontin and their use for the detection of an inflammatory disease, e.g. arthritis.

WO00/63247 discloses osteopontin-derived chemotactic peptide and inhibitory agents. Anti-osteopontin antibody is disclosed which recognizes LVLDPK site in the C-terminal side of osteopontin. It also discloses use of the inhibitory agents for treatment of autoimmune disease and inflammatory disease.

Various factors have been found to implicate in the pathogenesis of rheumatoid arthritis. Thus, many reports have been issued therefor. However, not any of them is reliable. Further, currently known therapeutic methods thereof are nosotropic and have not been essentially satisfactory.

Hence, it has been strongly desired to definitely elucidate the pathogenesis of rheumatoid arthritis and provide more excellent therapeutics thereof. It is a purpose of the invention to solve such problems.

Further, rheumatoid arthritis is hardly discriminated from osteoarthritis. Therefore, it is another purpose of the invention to provide a diagnostic method thereof.

### Disclosure of the Invention

The inventors found that the OPN concentration in each of the articular cavity fluids of rheumatism patients and osteoarthritis patients was at a higher value. Additionally, the inventors found the increase of the ratio of the N-terminal fragment of the thrombin-cleavage type in the total OPN in rheumatism patients for the first time. Thus, the inventors speculated that OPN might be deeply involved in the onset of these diseases. As described in the following Reference Example, then, the inventors verified the findings at experiments using OPN knockout mice.

Further, the inventors prepared antibodies individually discriminatively recognizing the N-terminal fragment and the C-terminal fragment from the thrombin-cleaved OPN. Then, the inventors found at experiments with the antibodies that the N-terminal fragment of the thrombin-cleaved OPN was at a high concentration in the articular cavity fluids of patients with rheumatoid arthritis, in particular.

The inventors focused their attention to the co-presence of the RGD sequence and the sequence SVVYGLR both recognized by human-type integrin, in the N-terminal fragment at such high concentration as observed in patients with rheumatoid arthritis. Then, the inventors anticipated that an antibody simultaneously blocking both the sequences would inhibit the binding between OPN and integrin widely so the antibody might be effective for the therapeutic treatment of rheumatoid arthritis and osteoarthritis.

Further, OPN is distributed in kidney, placenta, ovary, brain, skin and the like, but is mainly expressed in bone tissue. The inventors considered that for the therapeutic treatment of rheumatoid arthritis, the binding between OPN and integrin would preferably be blocked by a method more specific to the OPN side. Because the diverse integrin species are involved in inflammation in a cooperative manner, then, the inventors considered that it would be effective to more widely block the binding to these diverse integrin species.

Therefore, the inventors prepared an antibody inhibiting the binding between the RGD sequence of human OPN and integrin and the binding between the SVVYGLR sequence of human OPN and integrin and then verified the effects thereof at experiments for cell adhesion and cell migration and the like. Further, the inventors recovered an antibody against synthetic peptides corresponding to such inner sequences of murine OPN, to examine the efficacy of such antibody as a therapeutic agent, using an arthritis-diseased model in mouse.

More specifically, because murine OPN has the sequences RGD and SLAYGLR recognizable by murine integrin, which are located at positions homologous to human OPN in terms of amino acid sequence, an antibody M5 was recovered as an antibody simultaneously blocking these sequences. It was verified that the binding of the antibody M5 with murine OPN and the thrombin digestion products thereof was inhibited by the peptide GRGDSP including the sequence RGD site and that the antibody M5 inhibited the migration of TNF-α-activated monocyte derived from murine spleen. It was also observed that the antibody M5 had an action to suppress bone damage when examined in a murine calvaria organ culture system. Further, it was confirmed that the antibody exhibited an apparent therapeutic effect, when administered to a murine collagen arthritis model.

The aforementioned results strongly suggest that the antibody simultaneously blocking the binding on the sequences RGD and SVVYGLR sites with human-type integrin inhibits the binding between OPN and integrin and is therefore effective for the therapeutic treatment of rheumatoid arthritis and that the antibody will possibly be effective not only for rheumatism such as juvenile articular rheumatism and chronic rheumatism, but also for psoriatic arthritis and psoriasis. Additionally, chronic rejections after organ transplantation characteristically involve vascular and bronchial occlusive disorders. The histological examination thereof suggests that, because activation of T cell and macrophage triggers generation of cytokine and growth factors and disorders of vascular endothelial cell and because proliferation of vascular smooth muscle cell elicits fibrogenesis and the like, vascular occlusion will then possibly occur (P. Freese et al., (2001) : Nephrol Dial Transplant, 16, 2401-2406/J. R. Waller et al., (2001): British Journal of Surgery, 88, 1429-1441/S. R. Lehtonen et al., (2001): Transplantation, 72, 1138-1144). A report tells that OPN has a function as a protein essential for macrophage activation and fibrogenesis of vascular smooth muscle cell. (A. O'Regan et al., (2000) : Int J Exp Pathol, 81, 373-390). Thus, the inventive OPN inhibitory antibody of the invention suppresses migration of monocyte and neutrophil, thereby possibly suppressing a course toward such fibrogenesis. Thus, the antibody suppresses chronic rejections after organ transplantation, with the resultant contributions to organ adhesion. Additionally, the antibody will be effective for the therapeutic treatment of autoimmune diseases including systemic autoimmune diseases, erythematodes, uveitis, Behcet disease, multiple myositis, proliferative glomerulonephritis, sarcoidosis and the like.

In other words, the invention provides an anti-osteopontin antibody produced by the hybridoma FERM BP-7883, and inhibiting the binding between an integrin recognizing the sequence RGD and OPN or a fragment thereof and also widely inhibiting the binding between an integrin recognizing the sequence SVVYGLR or a corresponding sequence and osteopontin or a fragment thereof.

Further, the invention provides a therapeutic agent of rheumatism and a therapeutic agent of rheumatoid arthritis and a therapeutic agent for treating osteoarthritis, and these therapeutic agents contain the anti-osteopontin antibody as the effective ingredients.

Still further, the invention provides a use of the antibody for the manufacture of a medicament for treating rheumatism, rheumatoid arthritis and osteoarthritis.

### Brief Description of Drawings

Fig. 1 shows graphs depicting the inhibition of RGD-dependent cell adhesion to OPN.
Fig. 2 shows graphs depicting the inhibition of RGD-dependent and RGD-independent cell adhesion between nOPN and α9-transformed SW480 cell via the antibody 2K1.
Fig. 3a shows graphs depicting OPN-induced cell migration.
Fig. 3b shows graphs depicting the suppression of OPN-induced cell migration via antibodies.
Fig. 4 shows graphs depicting the time course of the change of arthritis score when the arthritogenic antibody cocktail/LPS was dosed individually to an OPN gene-defective mouse and normal mouse.
Fig. 5 shows graphs depicting the comparison of wrist swelling when the arthritogenic antibody cocktail/LPS was dosed to the OPN gene-defective mouse and normal mouse, individually.
Fig. 6 shows graphs depicting the adhesion between murine OPN and NIH3T3 in a concentration-dependent manner.
Fig. 7 shows graphs depicting the inhibition of the adhesion between murine OPN and NIH3T3 via the peptide GRGDSP.
Fig. 8 shows graphs depicting the inhibition of the adhesion between murine OPN and NIH3T3 via the antibody M5.

### Mode for Carrying out the Invention

The anti-osteopontin antibody (referred to as "OPN inhibitory antibody" hereinbelow) inhibiting the binding between an integrin recognizing the sequence RGD and OPN or a fragment thereof and also inhibiting the binding between an integrin recognizing the sequence SVVYGLR or a corresponding sequence and OPN or a fragment thereof is an antibody inhibiting the binding of an integrin recognizing the sequence RGD, for example αvβ1, αvβ3, and αvβ5 with OPN-a, or an N-terminal fragment thereof and also inhibiting the binding of an integrin recognizing the sequence SVVYGLR, for example α9β1, α4β1 and α4β7 with OPN-a, or an N-terminal fragment thereof. The sequence SVVYGLR or a corresponding sequence thereof means those described below: the sequence SVVYGLR means the sequence from serine at position 162 to arginine at position 168 in human OPN, while the corresponding sequence thereof means a SVVYGLR-corresponding sequence in the OPNs of other mammals, which is, for example, swine SVVYGLR identical to the sequence in humans, SVAYGLR in monkey, SLAYGLR in mouse and rat, SVAYGLK in bovine, and SVAYRLK in rabbit.

The method for preparing the antibody is not specifically limited. The OPN inhibitory antibody can be prepared by using for example OPN-a, or an N-terminal fragment thereof, or a peptide containing the amino acid sequence RGDSVVYGLR or a corresponding sequence thereof (referred to as "OPN-related peptide" hereinbelow) as the antigen. The OPN fragment herein referred to includes OPN fragments generated by digesting OPN with proteinases and the like, and is for example a fragment recovered by thrombin cleavage.

The OPN inhibitory antibody is preferably prepared by using a peptide containing the sequence RGDSVVYGLR as an antigen. More preferably, the OPN inhibitory antibody is prepared, for example, by using as an antigen the peptide (VDTYDGRGDSVVYGLRS) containing both the two sequences in a successive sequence, which starts from valine at position 153 and ends at serine at position 169 in the case of OPN-a, and subsequently treating the peptide according to a general method. In order to elevate the antigenicity, preferably, a product of the OPN-related peptide bound to a biopolymer compound is used.

For research works of OPN-related diseases, using mouse as an experimental animal, preferably, an OPN inhibitory antibody against murine OPN is used. Such antibody is preferably prepared by using a peptide containing the sequence RGDSLAYGLR as the antigen.

Examples of the biopolymer compound to be bound to the OPN-related peptide include for example Macroschisma hemocyanin (referred to as "KLH" hereinafter), ovalbumin (referred to as "OVA" hereinafter), bovine serum albumin (referred to as "BSA" hereinafter), rabbit serum albumin (referred to as "RSA" hereinafter), and thyroglobulin. Among them, KLH and thyroglobulin are more preferable.

The OPN-related peptide and the biopolymer compound are bound together by known methods, for example the mix acid anhydride process (B. F. Erlanger et al., (1954) : J. Biol. Chem. 234, 1090-1094) or the activated ester process (A. E. Karu et al., (1994): J. Agric. Food Chem. 42, 301-309).

The mix acid anhydride for use in the mix acid anhydride process can be recovered by subjecting the OPN-related peptide to general Schotten-Baumann reaction, which is then allowed to react with a biopolymer compound to prepare the object bound product of the peptide-polymer compound. The haloformate ester for use in the mix acid anhydride process includes for example methyl chloroformate, methyl bromoformate, ethyl chloroformate, ethyl bromoformate, isobutyl chloroformate and the like. The ratio of the peptide, the haloformate ester and the polymer compound to be used according to the method is appropriately selected in a wide range.

Herein, the Schotten-Baumann reaction is carried out in the presence of a basic compound. The basic compound for use in the reaction includes compounds for routine use for Schotten-Baumann reaction, for example organic bases such as triethylamine, trimethylamine, pyridine, dimethylaniline, N-methylmorpholine, diazabicyclononene (DBN), diazabicycloundecene (DBU), diazabicyclooctane (DABCO) and the like, and inorganic bases such as potassium carbonate, sodium carbonate, potassium hydrogen carbonate, sodium hydrogen carbonate and the like.

Additionally, the reaction is generally progressed at - 20 °C to 100 °C, preferably 0 °C to 50 °C. The reaction time is about 5 minutes to 10 hours, preferably 5 minutes to 2 hours.

The reaction between the resulting mix acid anhydride and the biopolymer compound is generally practiced at - 20°C to 150 °C, preferably 0 °C to 100 °C, for a reaction time of about 5 minutes to 10 hours, preferably 5 minutes to 5 hours. The mix acid anhydride method is generally carried out in a solvent. The solvent includes for example any of solvents commonly used for the mix acid anhydride method, specifically including halogenated hydrocarbons such as dichloromethane, chloroform, and dichloroethane; aromatic hydrocarbons such as benzene, toluene and xylene; ethers such as diethyl ether, dioxane, tetrahydrofuran, and dimethoxyethane; esters such as methyl acetate and ethyl acetate; non-protonic polar solvents such as N, N-dimethylformamide, dimethylsulfoxide, and hexamethylphosphotriamide; and the like.

Alternatively, the activation ester process is generally done as follows. Dissolving first the OPN-related peptide in an organic solvent, for reaction with N-hydroxysuccinimide in the presence of a coupling agent, an N-hydroxysuccinimide-activated ester is produced.

As the coupling agent, general coupling agents for routine use in condensation reaction can be used, including for example dicyclohexylcarbodiimide, carbonyldiimidazole and water-soluble carbodiimide. As the organic solvent, alternatively, for example, N,N-dimethylformamide (DMF), dimethylsulfoxide and dioxane can be used. The molar ratio of the peptide and a coupling agent such as N-hydroxysuccinimide for use in the reaction is preferably 1:10 to 10:1, most preferably 1:1. The reaction temperature is 0 °C to 50°C, preferably 22 °C to 27 °C, while the reaction time is 5 minutes to 24 hours, preferably one hour to 2 hours. Satisfactorily, the reaction temperature is a temperature above the individual melting points and below the individual boiling points.

After the coupling reaction, the reaction solution is added to a solution dissolving a biopolymer compound therein, for reaction. In the case that the biopolymer compound has a free amino group, for example, an acid-amide bond is formed between the amino group and the carboxyl group of the peptide. The reaction temperature is 0 °C to 60 °C, preferably 5 °C to 40 °C, and more preferably 22 °C to 27 °C, while the reaction time is 5 minutes to 24 hours, preferably one hour to 16 hours, and more preferably one hour to 2 hours.

The reaction product between the OPN-related peptide and the biopolymer compound as generated by the method is purified by dialysis or with a desalting column and the like, to recover the product of the OPN-related peptide bound to the biopolymer compound (simply referred to as "bound product" hereinafter)

Description now follows hereinbelow about the method for preparing an antibody, using the bound product thus recovered as an antigen, and an immunoassay method using the antibody. For the preparation of the antibody, herein, known methods can be utilized, appropriately, which are described in for example Zoku Seikagaku Jikken Koza (Biochemical Experimental Lecture Series), and Men-eki Seikagaku Kenkyu Ho (Immuno-Biochemistry Research Method) (Nihon Seikagaku Gakkai hen (Japan Biochemical Association, ed.)).

In order to prepare a polyclonal antibody using the bound product in accordance with the invention, an animal is immunized with the bound product to collect the antibody from the animal.

More specifically, for example, a bound product such as the OPN-related peptide-thyroglobulin bound product is first dissolved in sodium phosphate buffer (referred to as "PBS" hereinafter), which is then mixed with the Freund complete adjuvant or the Freund incomplete adjuvant, or an auxiliary agent such as alum. The resulting mixture is used as the immunogen for immunization of a mammalian animal.

Any animal for routine use in the field can be used as the animal for immunization, including for example mouse, rat, rabbit, goat and horse. Additionally, the method for dosing the immunogen for immunization may be via any of subcutaneous injection, intraperitoneal injection, intravenous injection, and intramuscular injection. Subcutaneous injection or intraperitoneal injection is preferable. Immunization can be done once or plural times at an appropriate interval, preferably at an interval of one week to 5 weeks.

According to a general method, then, blood is collected from the immunized animal, from which serum is separated. By purifying the polyclonal antibody fraction, the OPN inhibitory antibody can be recovered.

According to a general method, additionally, an immune cell recovered by immunizing an animal with the bound product is fused with myeloma cell to prepare a hybridoma. By collecting an antibody from a culture of the hybridoma, the OPN inhibitory antibody can be recovered as a monoclonal antibody.

When the antibody of the invention is intended for use in the therapeutic treatment of animals including humans, preference is given to the use of a chimera antibody (see European Patent Publication EP 0125023) prepared through such modification by genetic engineering that the resulting OPN inhibitory antibody might have the same constant region as that of such antibody for a subject human or animal to be treated or the use of the antibody having been animalized (see European Patent Publication EP 0239400 or EP 045126). Otherwise, preferably, a monoclonal antibody (animal-type antibody for the animal species) (see European Patent Publication EP 0546073 or WO 97/07671) is used, as prepared by using a transgenic animal with an artificially introduced gene involved in the generation of the antibody in a subject human or animal to be treated.

In the case that the subject to be treated is human and the OPN inhibitory antibody-generating animal is mouse, preferably, human mouse chimera antibodies or humanized antibodies are used. More preferably, a transgenic animal such as mouse introduced with a human gene involved in the antibody generation is used to prepare a human-type monoclonal antibody, for subsequent use. Further, the phage display method may satisfactorily be used for antibody generation.

The OPN inhibitory antibody thus recovered can be used in the form of Fv, Fab or F(ab')₂ with the antigen recognition site scissored out of the OPN inhibitory antibody with protease and the like.

The OPN inhibitory antibody thus recovered is further purified, if necessary, which is subsequently formulated into dosage forms according to a general method, for use in the therapeutic treatment of rheumatoid arthritis, rheumatism such as juvenile articular rheumatism and chronic rheumatism, or osteoarthritis.

The OPN inhibitory antibody of the invention can preferably be used as a therapeutic agent of rheumatism or a therapeutic agent of rheumatoid arthritis. Examples of the dosage forms of these therapeutic agents of rheumatism and the like include parenteral forms such as injections and infusions, which are preferably dosed via intravenous injection and subcutaneous injection (for use as a therapeutic agent of autoimmune diseases, the examples described above should be followed). For the formulation, additionally, pharmaceutically acceptable carriers and additives may be used within a pharmaceutically acceptable range, depending on the dosage form.

The amount of the OPN inhibitory antibody to be added for the formulation varies, depending on the symptomatic severity and age of a patient, the dosage form of the formulation to be used or the binding titer of the OPN inhibitory antibody or the like. For example, an amount of about 0.1 mg/kg to 100 mg/kg is satisfactorily used.
Because the OPN inhibitory antibody as the effective ingredient in the thus recovered therapeutic agent of the invention strongly binds to the sequences RGD and SVVYGLR in OPN, the OPN inhibitory antibody possibly inhibits the binding between these OPN regions and integrin, to consequently suppress the exacerbation of the symptoms of rheumatism, and rheumatoid arthritis and other autoimmune diseases.

Because the OPN inhibitory antibody of the invention specifically binds to the OPN side, not to the integrin side, the antibody potentially never inhibits other significant functions of integrin, so it is expected that disadvantageous side effects can be avoided.

### Examples

The invention will now be described in more detail in the following Examples and Reference Example.

### Example 1

Cloning, construction, purification and reagents for GST-OPN fusion protein:

Cloning and protein purification were done essentially according to the method described in the reference (S. Kon et al., (2000): J. Cell. Biochem. 77: 487-498).

The cDNAs of the human OPN isoforms i.e. OPN-a and OPN-b were recovered as follows. Using RNA prepared from NRC-12 cells of a human kidney cancer cell line as template, cDNA was synthetically prepared; using the cDNA as template, PCR was done using the following primers OPN-5 and OPN-3 to recover cDNAs encoding the full-length human OPN-a and OPN-b individually including the respective signal peptide regions.

In the manner as described in the reference, then, the thus cloned cDNAs of OPN-a and OPN-b were inserted in pGEX4T vector (Amersham Pharmacia Biotech, Tokyo, Japan) so that the cDNAs might be in the same reading frame as that of the GST gene (glutathione S-transferase; EC2.5.1.18), for expression in the form of GST fusion protein, using Escherichia coli JM109 (the GST-OPN fusion proteins thus recovered are referred to as "GST-OPN-a" and "GST-OPN-b" hereinbelow).
OPN-5:
   5'-CGGGATCCACTACCATGAGAATTGCAGTGATTTGC-3'
OPN-3:
   - 5'-CCGCTCGAGTTAATTGACCTCAGAAGATGCACTATC-3'

The cDNA encoding human OPN-c isoform was prepared by two-step PCR using the OPN-a cDNA as template. At a first step, PCR was individually done using OPN-5 and the following OPNct-3 primer or the following OPNct-5 and OPN-3 primer; the resulting two PCR products were mixed together, thermally treated, and gradually cooled for annealing, followed by addition of an enzyme for extension. At a second step, subsequently, PCR was done using the OPN-5 and OPN-3 primers, to recover cDNA encoding the full-length human OPN-c including the signal peptide region. The cDNA of the isoform c was integrated in pGEX4T vector by the same method as for the isoforms a and b, for preparing a GST fusion protein (referred to as "GST-OPN-c" hereinafter).
OPNct-3:
   5'-ACACAGCATTCTTTTCCACAGAACTTCCAGAATCAGC-3'
OPNct-5:
   5'-TGAGGAAAAGAATGCTGTGTCCTCTGAAGAAAACC-3'

The cDNA encoding the half moiety at the amino terminal side (M1-R168) from the thrombin-cleaved site of OPN-a was recovered by PCR using the OPN-a cDNA as template and OPN-5 together with the following OPNnh-3 primer described below. By the same method as for the isoforms a and b, the resulting cDNA was integrated in the pGEX4T vector to prepare a GST protein (referred to as "GST-Nhalf" hereinbelow).
OPNnh-3:
   5'-GCCTCGAGTTACCTCAGTCCATAAACCACACT-3'

The osteopontin protein (hOPN C half) on the carboxyl side from the thrombin-cleaved site of OPN-a was prepared by two-step PCR using the OPN-a cDNA as template. At a first step, PCR was done, individually, using OPN-5, the following OPNch-3 primer, the following OPNch-5 and the OPN-3 primer. At a second step, PCR was done using the OPN-5 and OPN-3 primers, to prepare the OPN protein on the carboxyl side. By the same method as for the isoforms a and b, recombination into pGEX4T vector enabled the preparation of a GST protein (referred to as "GST-Chalf" hereinafter).
OPNch-3:
   5'-TCTTAGATTTGGCACAGGTGATGCCTAGGAG-3'
OPNch-5:
   5'-CACCTGTGCCAAATCTAAGAAGTTTCGCAGA-3'

Various recombinant GST-OPN fusion proteins were prepared in Escherichia coli by a general method, and were then purified, using a glutathione-Sepharose column according to the method described in the reference. Among them, the GST-Nhalf protein was cleaved at the binding site with a prescission protease (PreScission; Amersham Pharmacia Biotech, Tokyo, Japan), to eliminate the GST protein moiety and thereby recover a protein (referred to as "nOPN" hereinafter) composed of the amino-terminal half moiety (I17-R168) of OPN alone.

Alternatively, the cDNA encoding the full-length OPN-a (M1-N314) was further inserted in pcDNA3.1 (+) vector (Invitrogen Corporation), for transfection into CHO-K1 cell (manufactured by Dainippon Pharmaceutical Co., Ltd.) (referred to as "CHO/OPN-a cell" hereinafter). The OPN-a of the sugar chain-bound type (referred to as "CHO/OPN-a" hereinafter) as recovered from the cell was purified as follows. Specifically, the culture supernatant of the CHO/OPN-a cell was subjected to ion exchange column chromatography using a DEAE-Sepharose CL-6B column (Amersham Pharmacia Biotech, Tokyo, Japan) and gel filtration chromatography on an Ultrogel AcA44 column (manufactured by BioSepra SA), and continuously to reverse-phase column chromatography on a RESOURCE RPC column (Amersham Pharmacia Biotech, Tokyo, Japan). In such manner, purification was completed.

Various peptides purchased from Sigma Genosis Japan were used for research works on immune sensitization and binding; otherwise, such peptides were recovered by chemical' synthesis by the Fmoc (N-(9-fluorenyl)methoxycarbonyl) process with a peptide synthesizer (Model 432 A; manufactured by PerkinElmer Life Science, Inc.) and purification by C18 reverse-phase column chromatography.

### Example 2

### Production of monoclonal antibody:

Synthetic peptides corresponding to the inner sequences of human OPN were prepared, as shown below, which were then used for immunization.
Peptide 1:
   CVDTYDGRGDSVVYGLRS (C+V153 to S169)
Peptide 2:
   CIDSQELSKVSREFHSH (C+I261 to H276)

Specifically, the Peptide 1 has the sequences RGD and SVVYGLR recognizing the αvβ3 and α9β1 integrin receptors, respectively.

These peptides were bound to thyroglobulin, which were then used for murine immunization according to a general method. Continuously, splenocytes were isolated from the immunizedmice, which were then subjected to cell fusion with a murine myeloma cell P3-X63-Ag8-653, using polyethylene glycol. According to the method described in the reference (M. Kinebuchi et al., (1991) : J. Immunol., 146, 3721-3728), a hybridoma reacting with each of the peptides used for the immunization was selected.

From mice immunized with the peptides 1 and 2 were recovered monoclonal antibodies designated 2K1 and 4C1, respectively. The hybridoma generating the monoclonal antibody 2K1 was deposited as FERMBP-7883 at the Patent Organism Depository Center, the National Institute of Advanced Industrial Science and Technology (AIST Tsukuba Central 6, 1-1-1, Higashi, Tsukuba-shi, Ibaraki 305-8566, Japan) on the date of June 20, 2001. Additionally, the monoclonal antibody 53 (mAb53) was recovered by immunization with the full-length recombinant human OPN (D. S. Bautista et al., (1994): J. Biol. Chem., 269, 23280-23285).

### Example 3

### Reactivity of OPN and thrombin digestion products thereof with the monoclonal antibodies:

The binding potencies of the monoclonal antibodies 2K1 and 4C1 recovered in the Example 2 to OPN and the thrombin digestion products thereof were tested by Western blotting method. It was found that the antibody 2K1 reacted with GST-OPN-a, GST-OPN-b, GST-OPN-c and GST-Nhalf. The antibody 4C1 reacted with GST-OPN-a, GST-OPN-b, GST-OPN-c and GST-Chalf. Further, these monoclonal antibodies were not only bound to the recombinant OPNs of sugar-chain-unbound type as generated in Escherichia coli but also reacted with the CHO/OPN-a protein of sugar-chain-bound type and the thrombin digestion products thereof (referred to as "thrombin-cleaved OPN" hereinafter).

### Example 4

### Inhibition of cell adhesion to OPN via the monoclonal antibodies:

It was examined by the following method as to whether or not the monoclonal antibodies inhabited cell adhesion to OPN. First, a 96-well plate was precoated with various concentrations of the CHO/OPN-a at 4 °C overnight, which was then treated with 0.5 % BSA in PBS under conditions of 37°C for 10 minutes, so as to block non-specific adhesion. A human fibroblast TIG-7 or SW480 cell transformed with the cDNA of an integrin subunit α9 (referred to as "α9-transformed SW480 cell" hereinafter) was suspended in D-MEM containing 0.25 % BSA; 200 µl of the resulting cell suspension (at a cell concentration of 5 × 10⁴ cells/well) was injected in a 96-well plate precoated with the CHO/OPN-a or nOPN, in the presence or absence of various concentrations of the monoclonal antibodies or synthetic peptides, for incubation at 37 °C for one hour.

The culture medium was discarded from the plate, and all the wells were rinsed twice with D-MEM containing 0.25 % BSA. The adherent cells were fixed and stained with 0.5 % crystal violet in 20 % methanol for 30 minutes.

All the wells were rinsed three times with water, and the adherent cells were then solubilized into 20 % acetic acid. The resulting supernatant recovered from each well was analyzed with an immunoreader, to measure the absorbance at 590 nm to determine the relative count of the cells adhering to the well. All the assays were done in a triplicate fashion, and at least three independent experiments were performed. The values shown represent mean from three independent experiments.

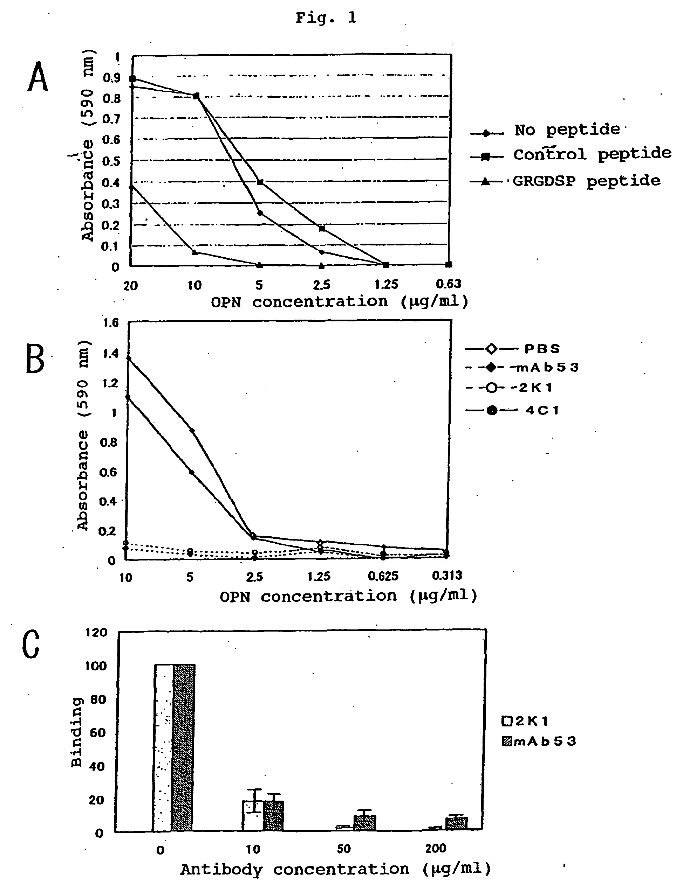

It has been known that TIG-7 highly adheres to OPN, but as shown in Fig. 1A, the adhesion is apparently inhibited by the peptide GRGDSP (100 µg/ml) but not inhibited by a control peptide (the C-terminal region of K296-N314 in OPN) (100 µg/ml). Thus, the adhesion is dependent on RGD. As shown in Fig. 1B, further, the antibody 2K1 at 200 µg/ml apparently inhibited the cell adhesion to OPN. As shown in Fig. 1C, still further, the effect of 2K1 on the inhibition of cell adhesion is comparative to the effect exerted by mAbs3 and is concentration-dependent. Still further, 2K1 and mAb53 never inhibit the adhesion of TIG-7 cell to vitronectin (VN) or fibronectin (FN).

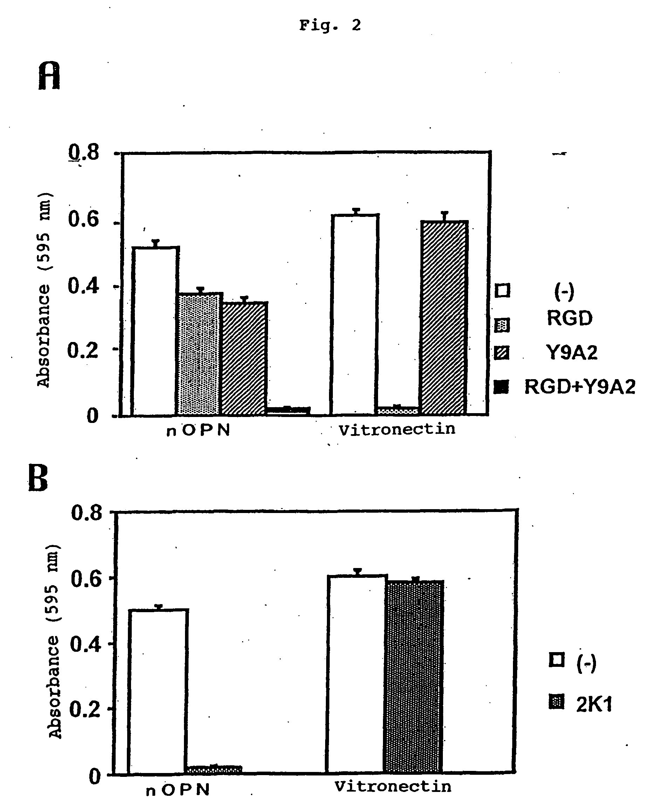

Fig. 2 depicts the inhibition of the monoclonal antibodies on the adhesion of nOPN and vitronectin to the α9-transformed SW480 cell. As shown in Fig. 2A, the adhesion between 1 µg/ml vitronectin and the α9-transformed SW480 cell was inhibited by 200 µM GRGDSP peptide(RGD), so the adhesion is dependent on the RGD. The adhesion of the α9-transformed SW480 cell to 3 µg/ml nOPN was inhibited by a combination of 200 µM GRGDSP and an anti-α9β1 monoclonal antibody Y9A2 (A. Wang et al., (1996): Am. J. Respir. Cell Mol. Biol., 15, 664-672), so the adhesion is RGD-dependent and RGD-independent. Fig.2B additionally shows the effect of 2K1 on the adhesion of the α9-transformed SW480 cell to nOPN and vitronectin. The adhesion between the α9-transformed SW480 cell and vitronectin was never inhibited by 2K1, but the adhesion between the SW480 cell and nOPN was inhibited by 2K1. Consequently, it is indicated that 2K1, retains the potency of inhibiting RGD-dependent adhesion.

### Example 5

### Inhibition of OPN-induced monocyte migration via the monoclonal antibodies:

Cell migration test using the U937 cell was performed by using a system ChemoTx101-8 (Neuro Probe Inc.). The cell was adjusted to 2 ×10⁶ cells/ml with D-MEM containing 0.1 % BSA, which was then applied to the upper layer on a filter (with a pore size of 8 µm), while the OPN protein was added to the lower layer.

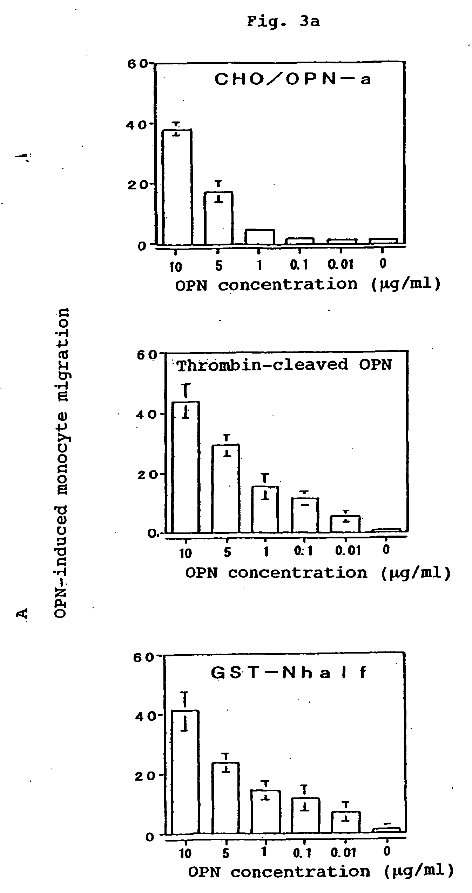

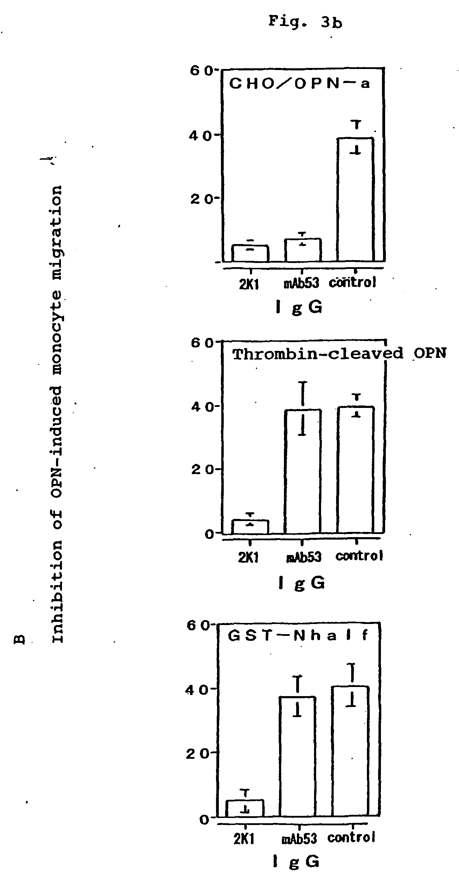

The ChemoTx plate was left to stand in the presence of 5 % CO₂ at 37 °C for 4 hours. After the plate was left to stand, the filter was fixed with methanol, and was then stained with hematoxylin and eosin (H-E). The number of the cells migrating into the back face of the filter was counted with a microscope (magnification × 400). The test was done in a triplicate manner, and the mean was used as data. The results are shown in Fig. 3.

Fig. 3a shows cell migration of the U937 cell toward the CHO/OPN-a, the thrombin-cleaved OPN and the GST-Nhalf at the concentrations shown. Additionally, Fig. 3b shows inhibition assays using the individual OPNs at 10 µg/ml, in the presence or absence of 50 µg/ml 2K1, mAb53 or control murine IgG after antigen-specific purification.

As shown in Figs. 3a and 3b, the CHO/OPN-a, the thrombin-cleaved OPN and the GST-Nhalf induce the migration of the human monocyte U937 in a concentration-dependent manner (A). The 2K1 antibody apparently inhibits the monocyte migration induced by the CHO/OPN-a, the thrombin-cleaved OPN and the GST-Nhalf. On contrast, mAb53 only inhibits the monocyte migration induced by the full-length OPN (B).

### Reference Example 1

### OPN and arthritis induction:

In order to elucidate the OPN function in arthritis, an OPN gene-defective mouse (S. R. Rittling et al., (1998) : J. Bone and Mminer. Res., 13 (7), 1101-1111) was artificially prepared according to a general method, for comparative experiments with normal mouse.

An arthritogenic monoclonal antibody cocktail commercially available as a substance eliciting arthritis (under the trade name of a cocktail for arthritis, Arthrogen-CIA^{®} mAb, Arthritogenic mAb cocktail; manufactured by Iwai Chemical Pharmaceutical Co., Ltd.) was administered to the OPN gene-defective mouse (OPN^{-/-}) and a normal mouse (OPN^{+/+}), individually, according to an instruction manual attached to the product, for arthritis induction. Then, the severity thereof was observed. For controls, physiological saline was dosed to the two types of the mice.

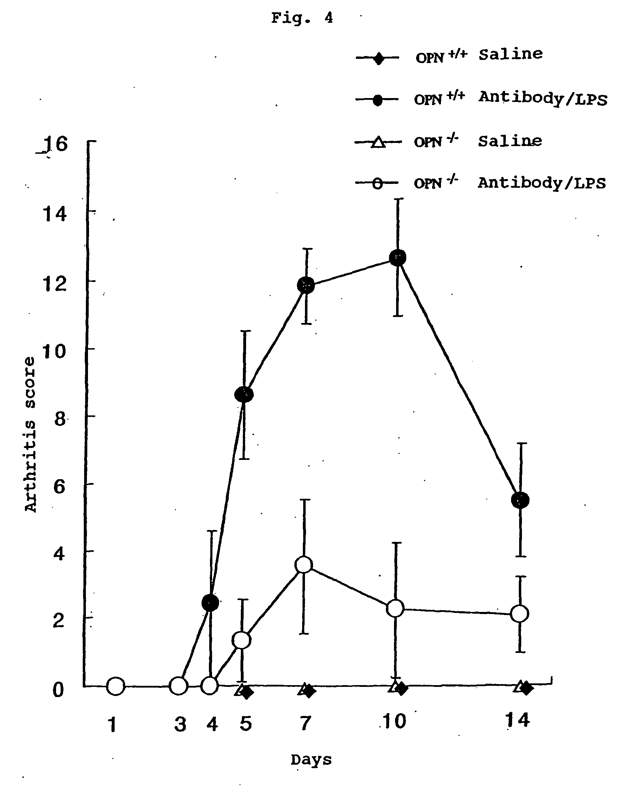

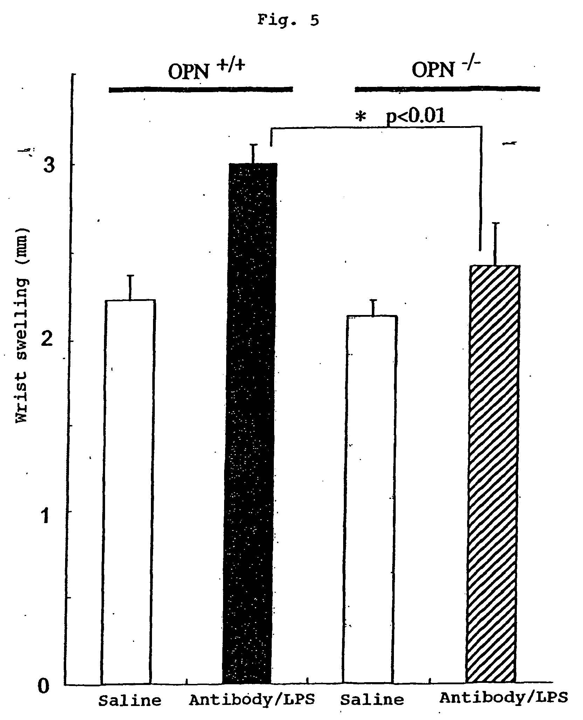

Comparison of the severity of arthritis was made on the basis of arthritis score according to the following standard and wrist swelling on day 10 post-dosing. The results are shown in Figs. 4 and 5.

As apparently shown in Fig. 4, the normal mouse dosed with the arthritogenic antibody cocktail/lipopolysaccharide (referred to as "LPS" hereinafter) had an increase of the arthritis score on day 4 and thereafter, until on day 10, the score reached maximum (12 or more). Alternatively, the arthritis score of the OPN gene-defective mouse increased on day 5 and thereafter, but the score was only 4 or less at maximum. Additionally, any of the groups dosed with physiological saline had no increase of the arthritis score.

As shown in Fig. 5, further, wrist swelling is apparently weak in the OPN gene-defective mouse, compared with the normal mouse, which clearly indicates OPN involvement in arthritis.

### Example 6

### Inhibitory activity of 2K1 antibody on human peripheral leukocyte migration:

By the following method, the inhibitory activity of the 2K1 antibody on cytokine-activated human peripheral leukocyte migration was examined. Table 1 shows the results of the inhibitory activity on neutrophil migration, while Table 2 shows the results of the inhibitory activity on monocyte migration.

### <Experimental method>

At the Ficoll process, a monocyte fraction and a neutrophil fraction were separated from normal human peripheral blood (P. M. Daftarian et al., (1996): Journal of Immunology, 157, 12-20). The intermediate layer between Ficoll and serum was collected and cultured in a flask at 37 °C for one hour. The resulting attached cell was used as monocyte. To the erythrocyte layer remaining after collection of the monocyte fraction was added a 5-fold volume of 3% dextran-PBS to aggregate erythrocyte, followed by centrifugation at 150 × g and 4°C for 5 minutes.

The aggregated erythrocyte was precipitated, while in the resulting supernatant, neutrophil existed in suspended state. Then, the fraction was centrifuged at 500 × g and ambient temperature for 20 minutes, to recover neutrophil. The monocyte and neutrophil as recovered in such manner were cultured overnight with human TNF-α (20 ng/mL) for activation. Then, the resulting activated monocyte and neutrophil were used for migration experiments.

The migration experiments were done, using a 48-well micro chemotaxis chamber (manufactured by Neuro Probe Inc.). After various concentrations of the 2K1 antibody were added to the thrombin-cleaved OPN and were then preliminarily left to stand at 37 °C for 15 minutes, the mixtures were added to the lower chamber (to a final human OPN concentration of 10 µg/mL). Placing thereon a polycarbonate filter (pore size of 5 µm), further, a cell suspension of 50 µL was added to the upper chamber (2 × 10⁶ cells/mL).

After culturing in the presence of 5 % CO₂ at 37 °C for 2 hours, the polycarbonate filter was removed to discard the cells on the upper surface of the filter; subsequently, the cells infiltrating into the back face of the filter were stained with Diff-Quick (manufactured by Baxter, International Inc.). The stained cells were counted at a magnification × 40. The results are shown as mean cell counts (cells/mm³) ± SD in 6 wells.

### <Experimental Results>

The 2K1 antibody inhibited the migration of the TNF-α-activated human peripheral neutrophil and monocyte toward the thrombin-cleaved OPN.

Inhibition of neutrophil migration:

**Table 1**

| Concentration of thrombin-cleaved OPN (µg/mL) | 2K1 concentration (µg/mL) | Mean migrating cell counts per 1 mm³ |
|---|---|---|
| 0 | 0 | 400.0 ± 67.8** |
| 10 | 0 | 581.7 ± 67.1 |
| 10 | 0.4 | 566.7 ± 60.2 |
| 10 | 2 | 550.0 ± 49.0 |
| 10 | 10 | 450.0 ± 90.8** |
| 10 | 50 | 426.7 ± 30.8** |

| | | |
|---|---|---|
| ** P<0.01(one way ANOVA, Dunnett's test) | | |

Inhibition of monocyte migration:

**Table 2**

| Concentration of thrombin-cleaved OPN (µg/mL) | 2K1 concentration (µg/mL) | Mean migrating cell counts per 1 mm³ |
|---|---|---|
| 0 | 0 | 58.3 ± 50.8** |
| 10 | 0 | 285.0 ± 49.3 |
| 10 | 0.4 | 258.0 ± 71.9 |
| 10 | 2 | 256.7 ± 66.5 |
| 10 | 10 | 160.0 ± 56.9** |
| 10 | 50 | 75.0 ± 55.4** |

| | | |
|---|---|---|
| ** P<0.01(one way ANOVA, Dunnett's test) | | |

### Example 7

### Preparation of M5 antibody:

The following synthetic peptide corresponding to the inner sequence (C+V138 to R153) of murine OPN was prepared for use in immunization.
M5 peptide:
   CVDVPNGRGDSLAYGLR

The peptide was bound to thyroglobulin, for subsequent use for rabbit immunization according to a general method. Anti-serum was collected from the immunized rabbit, to prepare the M5 antibody, using a column packed with the M5 peptide of the N terminal cysteine bound through disulfide bond to thiol Sepharose beads (Amersham Pharmacia Biotech, Tokyo, Japan).

### Example 8

### Reactivity of the M5 antibody with OPN and the thrombin digestion products thereof:

The binding potency of the M5 antibody recovered in Example 7 with OPN and the thrombin digestion products thereof was tested by the Western blotting method. Recombinant murine OPN of the glycosylated form as generated in CHO cells was used as the OPN. The M5 antibody reacted with OPN and the thrombin digestion products thereof.

### Example 9

### Inhibition of cell adhesion to OPN via the M5 antibody:

It was examined by the method described in the reference (S. kon et al., (2002) : J. Cell. Biochem., 84(2), 420-432) as to whether or not the M5 antibody might inhibit cell adhesion to OPN. As the OPN, the full-length murine OPN was used, from which the GST moiety had preliminarily been removed with PreScission protease (Amersham Pharmacia Biotech, Tokyo, Japan) (referred to as "mOPN/de-GST" hereinafter) was used. As the cell, murine NIH3T3 cell was used.

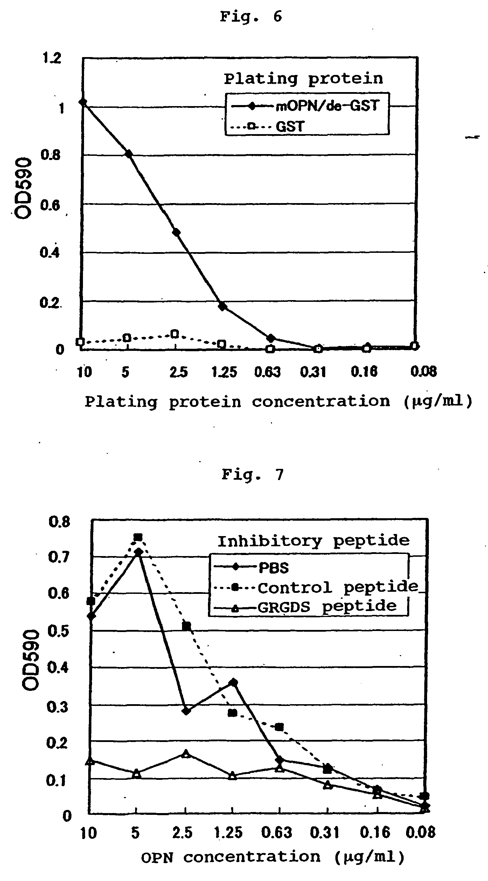

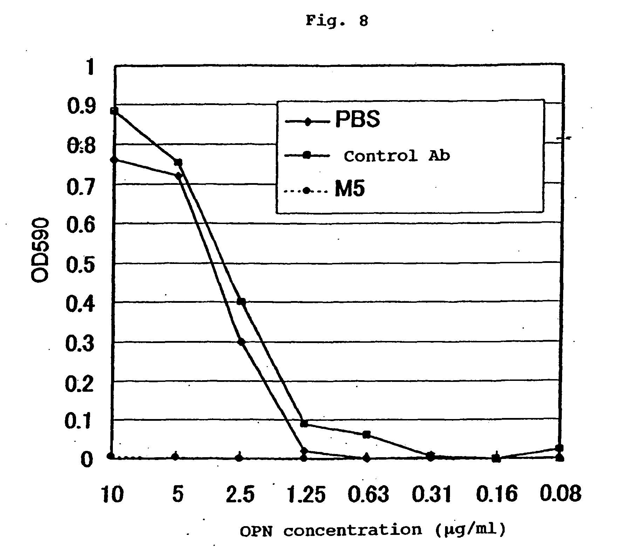

As shown in Fig. 6, the NIH3T3 cell adheres to mOPN/de-GST in a concentration-dependent manner. As shown in Fig. 7, further, the adhesion is apparently inhibited by the peptide GRGDSP (100 µg/mL), so the adhesion depends on RGD. As shown in Fig. 8, the M5 antibody at 200 µg/mL apparently inhibited cell adhesion to OPN.

### Example 10

### Inhibitory activity of the M5 antibody against murine spleen-derived monocyte migration:

The inhibitory activity of the M5 antibody against cytokine-adtivated murine spleen-derived monocyte migration was examined by the following method. The results are shown in Table 3.

### <Experimental method>

The splenocyte from C57BL/6 mouse was ground with a slide glass into a single cell, which was then cultured in a flask at 37 °C for one hour. The resulting adherent cell was used as monocyte. The monocyte was overnight cultured and activated with human TNF-α (20 ng/mL). The resulting activated monocyte was used at a migration experiment. The migration experiment was done by the same method as for the human sample in Example 6 above.

### <Experimental results>

The M5 antibody inhibited the migration of TNF-α-activated monocyte derived from murine spleen toward the thrombin-cleavage-type murine OPN recovered from cleavage of the full-length murine OPN (manufactured by Genzyme Corporation) with bovine thrombin (manufactured by Sigma).

**Table 3**

| Concentration of thrombin-cleavage-type murine OPN (µg/mL) | M5 concentration (µg/mL) | Mean migrating cell counts per 1 mm³ |
|---|---|---|
| 0 | 0 | 428.3 ± 52.7** |
| 10 | 0 | 556.7 ± 46.3 |
| 10 | 0.8 | 570.0 ± 75.6 |
| 10 | 4 | 536.7 ± 60.6 |
| 10 | 20 | 461.7 ± 104.4 |
| 10 | 100 | 468.3 ± 67.9 |

| | | |
|---|---|---|
| ** P<0.05 (one way ANOVA, Dunnett's test) | | |

### Example 11

### Action of the M5 antibody on bone damage suppression:

By the following method, the action of the M5 antibody on bone damage suppression in a murine calvaria organ culture system was examined. The results are shown in Table 4.

### <Experimental method>

From a newborn mouse on day 1 after birth was resected the cranial bone; after size adjustment, a half thereof was placed in each well of a 24-well plate. To each well was then added human parathyroid hormone (PTH) (1-34) adjusted with addition of a D-MEM culture broth (with 10 % bovine serum added) to a final PTH concentration of 10 nM, to induce bone damage. The M5 antibody was added to a final concentration of 200 µg/mL. After culturing at 37 °C for one week, the quantity of calcium released from bone into the culture broth was assayed by Calcium E Test WAKO (Wako Pure Chemical Industries, Ltd.).

### <Experimental results>

With no PTH addition, calcium was at a value of 7.02 mg/mL. With PTH addition, however, calcium was at a value of 9.11 mg/mL. Hence, it was indicated that calcium release from bone was promoted. When the M5 antibody was added at a concentration of 200 µg/mL, it was verified that bone absorption was inhibited by about 70 %.

**Table 4**

| | Quantity of calcium released (mg/dL) |
|---|---|
| Medium | 7.02 ± 0.18** |
| PTH control | 9.11 ± 0.17 |
| M5 | 7.65 ± 0.25** |

| | |
|---|---|
| ** P<0.01(one way ANOVA, Dunnett's test) | |

### Example 12

### Effect of M5 antibody in mouse collagen arthritis model:

By the following method, the effect of the M5 antibody in a mouse collagen arthritis model was examined. Table 5 shows the results about arthritis score; Table 6 shows the results about leg edema; Table 7 shows the results about body weight change; and Table 8 shows the results about the change of feed intake.

### <Experimental method>

For arthritis induction, an arthritogenic antibody cocktail (under the trade name of a cocktail for arthritis, Arthrogen-CIA^{®} mAb, Arthritogenic mAb cocktail; manufactured by Iwai Chemical Pharmaceutical Co., Ltd.) recognizing four epitopes specific to collagen was used. To a mouse was intravenously administered the arthritogenic cocktail; 3 days later, LPS (100 µg) was intraperitoneally administered to thereby elicit arthritis. Arthritis was observed on day 3 after LPS dosing, which reached maximum on day 6.

Immediately before LPS dosing and 3 days later, the M5 antibdy was intravenously administered at a dose of 40 µg, 150 µg or 400 µg. As the control group, a rabbit IgG-dosed group (at a dose of 400 µg) was arranged. Additionally, the anti-mouse TNF-α antibody was intravenously administered at a dose of 200 µg/mouse immediately before LPS administration and 3 days later. As the control group, a rat IgG-dosed group (at a dose of 200 µg) was arranged. Further, MTX was orally given (at a dose of 3.2 mg/kg) once daily on the very day of LPS dosing and thereafter. Then, MTX dissolved in 5 ml of 0.5 % methyl cellulose solution was used. For the control group, 5 ml of 0.5 % methyl cellulose solution was arranged.

Assessment was done per one group of five animals, concerning four items, namely arthritis score, leg edema, body weight change and feed intake.

### <Experimental results>

As shown in Tables 5 to 8, the M5 antibody exerted distinct suppressive actions on the improvement of arthritis score, the delay of the onset of arthritis, and the improvement of leg edema in the mouse arthritis model (therapeutic effect). The onset of arthritis was suppressed in a concentration-dependent manner at a level such that the effect exceeded the effect of the anti-mouse TNF-α antibody dosed (at a dose of 200 µg/mouse). In contrast, MTX exerted almost no pharmaceutical efficacy.

In the normal group of the model, furthermore, distinct body weight decrease by about 3 g was observed in 3 days after LPS dosing; and the tendency was continued on day 3 to day 6, although the decrease ratio was more or less reduced. In the groups dosed with the M5 antibody (150 µg, 400 µg) and the group dosed with the anti-mouse TNF-α antibody, alternatively, apparent improvement of body weight decrease was observed. Concerning the feed intake, furthermore, rapid body weight decrease was observed for any of the pharmaceutical substances up to day 3 after LPS dosing; on day 3 to day 6, however, the decrease was improved in the groups dosed with the M5 antibody and the group dosed with the anti-mouse TNF-α antibody. Table 5 shows the effect on arthritis score; Table 6, the suppressive effect on leg edema; and Tables 7 and 8 show the effects on body weight change and feed intake change, respectively.

**Table 5**

| Days | Rabbit IgG-dosed group | M5 antibody-dosed group(dose;µg/mouse) | | |
|---|---|---|---|---|
| | (dose;400µg/mouse) | 40 | 150 | 400 |
| 3 | 1.2±1.1 | 2.4±1.7 | 1.0±1.2 | 0.0±0.0 |
| 4 | 1.8±1.3 | 3.4±1.1 | 1.4±0.5 | 0.2±0.4* |
| 5 | 5.0±1.6 | 6.0±2.0 | 3.0±1.2* | 1.8±0.4** |
| 6 | 5.4±1.3 | 7.2±1.3 | 4.6±1.5 | 3.0±1.0* |

| Days | Rat IgG-dosed group (dose;200µg/mouse) | Anti-mouse TNF-α antibody-dosed group (dose;200µg/mouse) | | |
|---|---|---|---|---|
| 3 | 0.8±0.4 | 1.0±0.6 | | |
| 4 | 2.4±0.2 | 1.0±0.5 | | |
| 5 | 5.8±0.4 | 3.2±0.5* | | |
| 6 | 6.6±0.4 | 5.8±0.5 | | |

| Days | Control group | MTX-dosed group (dose;3.2mg/kg) | | |
|---|---|---|---|---|
| 3 | 1.0±0.3 | 0.8±0.4 | | |
| 4 | 1.8±0.6 | 1.8±0.7 | | |
| 5 | 4.0±0.9 | 6.0±0.6 | | |
| 6 | 5.2±0.9 | 6.0±0.6 | | |

| | | | | |
|---|---|---|---|---|
| ** P<0.01, *P<0.05 (non-parametric Mann-Whitney test) * P<0.05(non-parametric Mann-Whitney test) | | | | |

**Table 6**

| | Volume of leg edema (mL) | | | | |
|---|---|---|---|---|---|
| Site | Normal group | Rabbit IgG-dosed group | M5-dosed group (dose;µg/mouse) | | |
| | | (dose; 400µg/mouse) | 40 | 150 | 400 |
| Foreleg | 0.041±0.003** | 0.051±0.004 | 0.055±0.004 | 0.041±0.004** | 0.038±0.001** |
| Hind leg | 0.117±0.004** | 0.138±0.005 | 0.140±0.010 | 0.127±0.006 | 0.121±0.009** |

| | Volume of leg edema (mL) | | | | |
|---|---|---|---|---|---|
| Site | Normal group | Rat IgG-dosed group | Anti-mouse TNF-α antibody-dosed group | | |
| | | (dose; 200µg/mouse) | (dose;200µg/mouse) | | |
| Foreleg | 0.041±0.003** | 0.044±0.003 | 0.041±0.002 | | |
| Hind leg | 0.117±0.004** | 0.127±0.004 | 0.130±0.006 | | |

| | Volume of leg edema (mL) | | | | |
|---|---|---|---|---|---|
| Site | Normal group | Control group | MTX-dosed group | | |
| | | | (dose;3.2mg/kg) | | |
| Foreleg | 0.041±0.003** | 0.044±0.002 | 0.047±0.003 | | |
| Hind leg | 0.117±0.004** | 0.129±0.005 | 0.132±0.003 | | |

| | | | | | |
|---|---|---|---|---|---|
| ** P<0.01, *P<0.05 (one way ANOVA, Dunnett's test) | | | | | |

**Table 7**

| | Body weight change (g) | | | | |
|---|---|---|---|---|---|
| | Normal group | Rabbit IgG-dosed group | M5-dosed | group(dose;µg/mouse) | |
| | | (dose;400µg/mouse) | 40 | 150 | 400 |
| On day 0 to day 3 post-dosing | 0.1 | -2.8 | -2.4 | -1.6 | -1.5 |
| On day 3 to day 6 post-dosing | 0.5 | 1.8 | 1.0 | 0.8 | 1.5 |

| | Body weight change (g) | | | | |
|---|---|---|---|---|---|
| | Normal group | Rat IgG-dosed group | Anti-mouse TNF-α antibody-dosed group | | |
| | | (dose;200µg/mouse) | (dose;200µg/mouse) | | |
| On day 0 to day 3 post-dosing | 0.1 | -2.9 | -1.7 | | |
| On day 3 to day 6 post-dosing | 0.5 | -1.7 | -0.1 | | |

| | Body weight change (g) | | | | |
|---|---|---|---|---|---|
| | Normal group | Control group | MTX-dosed group | | |
| | | | (dose;3.2mg/kg) | | |
| On day 0 to day 3 post-dosing | 0.1 | -2.9 | -1.5 | | |
| On day 3 to day 6 post-dosing | 0.5 | -2.7 | -1.8 | | |

**Table 8**

| | Quantity of feed intake (g/mouse/day) | | | | |
|---|---|---|---|---|---|
| | Normal group | Rabbit IgG-dosed group | M5-dosed group(dose;µg/mouse) | | |
| | | (dose;400µg/mouse) | 40 | 150 | 400 |
| On day 0 to day 3 post-dosing | 2.7 | 1.0 | 0.9 | 1.4 | 1.1 |
| On day 3 to day 6 post-dosing | 2.9 | 2.4 | 2.3 | 2.4 | 2.8 |

| | Quantity of feed intake (g/mouse/day) | | | | |
|---|---|---|---|---|---|
| | Normal group | Rat IgG-dosed group | Anti-mouse TNF-α antibody-dosed group (dose;200µg/mouse) | | |
| | | (dose;200µg/mouse) | | | |
| On day 0 to day 3 post-dosing | 2.7 | 1.0 | 1.3 | | |
| On day 3 to day 6 post-dosing | 2.9 | 2.5 | 2.8 | | |

| | Quantity of feed intake (g/mouse/day) | | | | |
|---|---|---|---|---|---|
| | Normal group | Control group | MTX-dosed group | | |
| | | | (dose;3.2mg/kg) | | |
| On day 0 to day 3 post-dosing | 2.7 | 0.9 | 0.8 | | |
| On day 3 to day 6 post-dosing | 2.9 | 2.4 | 2.1 | | |

### Example 13

### Availability of OPN-related fragment peptides:

OPN-related fragment peptides at a state purified by HPLC chromatography were purchased from Auspep Inc., Parkiville, Australia. The amino acid sequences thereof are shown in (1) to (3).
hOPN5:
   CVDTYDGRGDSVVYGLRS (C+V153 to S169) (1)
hOPN3:
   KSKKFRRPDIQYPDATDEC (K170 to E187+C) (2)
hOPN1:
   IPVKQADSGSSEEKQC (I17 to Q31+C) (3)

### Example 14

### Preparation of antigens for immunization:

Products of the OPN-related fragment peptides bound to thyroglobulin were prepared by the EMCS (N-(6-maleimidocaproyloxy)-succinimide) process, as follows. For preparation of such products, the molar ratio of thyroglobulin, an OPN-related fragment peptide and EMCS was 1:300:400.

4 mg of each of the OPN-related fragment peptides in Example 13 was dissolved in distilled water of about 1 ml. Alternatively, 5 mg thyroglobulin dissolved in 1 ml of 0.01 M phosphate buffer, pH 7.0 and EMCS dissolved at 80 µg/µl in dimethylformamide were mixed together, individually at quantities corresponding to the moles, to prepare a thyroglobulin-EMCS complex solution. The complex solution was divided in three portions. To each of the portions was added the OPN-related fragment peptide solution at a quantity corresponding to the mole, to thereby prepare a solution of an EMCS-crosslinked product of the OPN-related fragment peptide bound to thyroglobulin.

The solution of such bound product was dialyzed, using PBS, to adjust the concentration of the product to 10 µg/µl. The bound product of the OPN-related fragment peptide and thyroglobulin was used as an antigen for immunization.

### Example 15

### Preparation of antigens for screening:

As OPN-proteins for screening, fusion proteins between GST and human OPN isoforms, namely GST-OPN-a, GST-OPN-b and GST-OPN-c, and fusion proteins between GST and the OPN fragment on the side of amino group (GST-Nhalf) from the thrombin cleavage site and the OPN fragment on the side of carboxyl group (GST-Chalf) from the same thrombin cleavage site were prepared by the method described in Example 1, for use in the anti-serum reactivity with OPN.

### Example 16

### Immune sensitization:

Rabbit was immunized, using as antigens for immunization, the bound products of the OPN-related fragment peptides and thyroglobulin prepared in Example 14. Immunization was done, by boosting 100 µl (100 µg) of a bound product solution every one week or every two weeks. The antigens were mixed with the Freund complete adjuvant for a first immunization and were then mixed with the Freund incomplete adjuvant for a second immunization and the following immunizations. After eight times of immunization, serum was separated from collected blood, which was then used as anti-serum.

### Example 17

### Reactivity of anti-serum with OPN:

The OPN-related fragment peptides prepared in Example 13 were diluted with 0.1 M carbonate buffer, pH 9.5 to 10 µg/ml, which were then immobilized at 50 µl/well on a 96-well plate. After rinsing with PBS and blocking with 0.1 % BSA/PBS/0.05 % NaN₃ solution, a 2-fold serial dilution of the 100-fold dilution of the anti-serum recovered in Example 16 was placed at 50 µl in a well, for reaction at 37 °C for 30 minutes.

After termination of the reaction, the well was rinsed four times with 0.05 % Tween 20-PBS. Then, 50 µl each of HRP-labeled anti-rabbit IgG (manufactured by IBL Co., Ltd.) was added to each well, for reaction at 37 °C for 30 minutes. After termination of the reaction, 100 µl each of 0.05 M citrate buffer, pH 4.5 containing 0.4 mg/ml orthophenylenediamine (OPD) and aqueous 0.03 % hydrogen peroxide was added to each well. Then, the plate was left to stand in darkness at ambient temperature for 15 minutes, for chromogenic reaction. After the chromogenic reaction, 100 µl of 1N sulfuric acid was added to each well, to terminate the reaction, for absorbance measurement at 492 nm.

Using the OPN proteins prepared in Example 15, alternatively, the reactivity of anti-serum was examined by Western blotting method. Consequently, anti-sera against the OPN-related fragment peptides hOPN1 and hOPN5 reacted with GST-OPN-a, GST-OPN-b, GST-OPN-c and GST-Nhalf, but never reacted with GST-Chalf. Alternatively, anti-serum against the OPN-related fragment peptide hOPN3 reacted with GST-OPN-a, GST-OPN-b, GST-OPN-c and GST-Chalf, but never reacted with GST-Nhalf.

### Example 18

### Preparation of HRP-bound products of anti-OPN-related fragment peptides antibodies:

HRP-bound products of the antibodies against the OPN-related fragment peptides hOPN3 and hOPN1 were prepared as follows. 20 mg of each anti-OPN-related fragment peptide antibody was digested with pepsin, followed by gel filtration to purify the F(ab')₂ fragment of the anti-OPN-related fragment peptide antibody. Then, the F(ab')₂ fragment was reduced to Fab' fragment, by using 2-mercaptoethanol. HRP reacted with EMCS at 37 °C for 60 minutes, followed by gel filtration to prepare a HRP-EMCS bound product, which further reacted with the anti-OPN-related fragment peptide antibody Fab' fragment at 4 °C overnight, followed by gel filtration to prepare an EMCS-crosslinked HRP-bound product of the anti-OPN-related fragment peptide antibody.

### Example 19

### Construction of sandwich ELISA systems:

From combinations of a sandwich ELISA plate and labeled antibodies, two types of systems namely 1-3 and 5-1 were prepared. Specifically, the 1-3 system was prepared as follows. The 10 µg/ml antibody against the OPN-related fragment peptide hOPN1 was added in 100 µl portions to a 96-well ELISA plate. After overnight reaction at 4 °C, blocking with 10 % BSA/PBS/NaN₃ solution was done. The resulting plate at that state was used as the sandwich ELISA plate. The HRP-bound product of the antibody against the OPN-related fragment peptide hOPN3 prepared in Example 18 was defined as labeled antibody. As described above, a combination between the immobilizing plate using the antibody against hOPN1 and the labeled antibody using the antibody against hOPN3 was defined as system 1-3.

In the same manner, a combination of an immobilizing plate using the antibody against hOPN5 and a labeled antibody using the antibody against hOPN1 was constructed as system 5-1.

### Example 20

### Osteopontin assay in a test subject by sandwich ELISA systems:

The OPN protein was assayed as follows. 100 µl of a solution containing a plasma sample or an articular cavity fluid sample from a test subject was added to the sandwich ELISA plates of the systems 1-3 and 5-1, for reaction at 37 °C for one hour. After reaction, the plates were rinsed four times with 0.05 % Tween20-PBS, followed by addition of 100 µl each of the labeled antibodies specific to the individual systems for reaction at 4 °C for 30 minutes. After reaction, the plates were rinsed six times with 0.05 % Tween20-PBS, followed by addition of 100 µl of a TMB (tetramethylbenzidine) solution. Then, the resulting plates were left to stand in darkness at ambient temperature for 30 minutes. 1N sulfuric acid was used to terminate the reaction, for the assay of the absorbance at 450 nm.

Table 9 shows the OPN values in the articular cavity fluids of patients (13 cases) with rheumatism as measured by the method; and Table 10 shows the OPN values in the articular cavity fluids of patients (12 cases) with osteoarthritis. Additionally, Table 11 shows the OPN values in the plasmas from rheumatism patients (16 cases) ; Table 12 shows the OPN values in the plasmas from osteoarthritis patients (7 castes) ; and Table 13 shows the OPN values in the plasmas from normal subjects (6 cases).

As apparently shown in these results, the comparison with the system 1-3 in terms of plasma OPN value among the patients with rheumatoid arthritis, the patients with osteoarthritis, and the normal subjects did not show any significant difference. However, the comparison with the system 5-1 in terms of plasma OPN value among the patients with rheumatoid arthritis, the patients with osteoarthritis, and the normal subjects showed significantly higher plasma OPN values in the patients with rheumatoid arthritis and the patients with osteoarthritis than the OPN value in the normal subjects. The level of significance was higher in the patients with rheumatoid arthritis. This indicates that total OPN quantity reflected with the system 5-1 is effective for the diagnosis of the general category of arthritis.

Alternatively, OPN values in the articular cavity fluids of the patients with rheumatoid arthritis and the patients with osteoarthritis are larger than the OPN values in the plasmas thereof, which strongly indicates local OPN generation.

Additionally, the comparison with the systems 1-3 and 5-1 in terms of OPN value of articular cavity fluid between the patients with rheumatoid arthritis and the patients with osteoarthritis showed that the OPN value in the patients with rheumatoid arthritis was significantly larger than the OPN value in the patients with osteoarthritis, when any of the systems 1-3 and 5-1 was used.

As a new indicator, the ratio of OPN values with the systems 1-3 and 5-1 was examined. The indicator can be used for the comparison of the ratio of the thrombin-cleaved OPN. The OPN values in the plasmas and articular cavity fluids from rheumatoid arthritis patients were 1 or less, and the OPN values from osteoarthritis patients were 2 or more, so significant difference was observed. Thus, the OPN values with the systems 1-3/5-1 can be used for a diagnostic method for discriminating rheumatoid patients from osteoarthritis patients at an early stage.

**Table 9**

| Sample | System I-3 (ng/ml) | System 5-I (ng/m) | System I-3 /system 5-I |
|---|---|---|---|
| RA 1 | 8498 | 2932 | 2.898 |
| RA 2 | 22715 | 26223 | 0.866 |
| RA 3 | 2659 | 1905 | 1.396 |
| RA 4 | 20186 | 94430 | 0.214 |
| RA 5 | 1520 | 2002 | 0.759 |
| RA 6 | 5870 | 2238 | 2.623 |
| RA7 | 7303 | 56753 | 0.129 |
| RA 8 | 2200 | 6268 | 0.351 |
| RA 9 | 18344 | 59873 | 0.306 |
| RA10 | 2133 | 2002 | 1.065 |
| RA11 | 26804 | 33036 | 0.811 |
| RA12 | 18868 | 32824 | 0.575 |
| RA13 | 3633 | 6067 | 0.599 |
| Mean | 10825.6 | 25119.5 | 0.969 |

**Table 10**

| Sample | System I-3 (ng/ml) | System 5-I (ng/ml) | System I-3 /system 5-I |
|---|---|---|---|
| OA1 | 1520 | 3471 | 0.438 |
| OA 2 | 9957 | 14374 | 0.693 |
| OA3 | 6595 | 2932 | 2.249 |
| OA4 | 3523 | 237 | 14.865 |
| OA5 | 8619 | 28483 | 0.303 |
| OA6 | 1926 | 896 | 2.150 |
| OA7 | 653 | 850 | 0.768 |
| OA8 | 6490 | 7814 | 0.831 |
| OA9 | 4750 | 1987 | 2.391 |
| OA10 | 6830 | 2932 | 2.329 |
| OA11 | 386 | 181 | 2.133 |
| OA12 | 1621 | 356 | 4.553 |
| Mean | 4405.8 | 5376.1 | 2.808 |

**Table 11**

| Sample | System I-3 (ng/ml) | System 5-1 (ng/ml) | System 1-3 /system-5-I |
|---|---|---|---|
| RA 1 | 1621 | 1379 | 1.175 |
| RA 2 | 532 | 845 | 0.630 |
| RA 3 | 132 | 617 | 0.214 |
| RA 4 | 142 | 1758 | 0.081 |
| RA 5 | 624 | 2089 | 0.299 |
| RA 6 | 341 | 1990 | 0.171 |
| RA 7 | 152 | 845 | 0.180 |
| RA 8 | 671 | 224 | 2.996 |
| RA 9 | 543 | 557 | 0.975 |
| RA10 | 947 | 431 | 2.197 |
| RA11 | 935 | 1794 | 0.521 |
| RA12 | 1008 | 1650 | 0.611 |
| RA13 | 636 | 678 | 0.938 |
| RA14 | 464 | 545 | 0.851 |
| RA15 | 683 | 488 | 1.400 |
| RA16 | 1057 | 597 | 1.771 |
| Mean | 6555 | 1030.4 | 0.938 |

**Table 12**

| Sample | System I-3 (ng/ml) | System 5-I (ng/ml) | System I-3 /system 5-1 |
|---|---|---|---|
| OA1 | 695 | 302 | 2.301 |
| OA2 | 1094 | 412 | 2.655 |
| OA3 | 1070 | 557 | 1.921 |
| OA4 | 75 | 1129 | 0.066 |
| OA5 | 814 | 356 | 2.286 |
| OA6 | 959 | 276 | 3.475 |
| OA7 | 983 | 311 | 3.161 |
| Mean | 812.9 | 477.6 | 2.267 |

**Table 13**

| Sample | System I-3 (ng/ml) | System 5-I (ng/ml) | System I-3 /system 5-1 |
|---|---|---|---|
| Normal1 | 475 | 199 | 2.387 |
| Normal 2 | 578 | 249 | 2.321 |
| Normal 3 | 802 | 232 | 3.457 |
| Normal 4 | 983 | 384 | 2.560 |
| Normal 5 | 520 | 284 | 1.831 |
| Normal 6 | 624 | 215 | 2.902 |
| Mean | 663.7 | 260.5 | 2.576 |

## Claims

1. An anti-osteopontin antibody produced by the hybridoma deposited as FERM BP-7883.

2. An anti-osteopontin antibody which is produced by humanization of the antibody of claim 1.

3. A therapeutic agent for treating rheumatism , rheumatoid arthritis or osteoarthritis comprising the anti-osteopontin antibody according to any one of claims 1 to 2 as an active ingredient.

4. Use of the anti-osteopontin antibody according to any one of claims 1 to 2 for the manufacture of a medicament for treating rheumatism, rheumatoid arthritis or osteoarthritis.

## Patentansprüche

1. Anti-Osteopontin-Antikörper, der von dem Hybridom gebildet wird, das als FERM BP-7883 hinterlegt ist.

2. Anti-Osteopontin-Antikörper, der durch Humanisierung des Antikörpers nach Anspruch 1 gebildet wird.

3. Therapeutisches Mittel zur Behandlung von Rheumatismus, rheumatoider Arthritits oder Osteoarthritis, umfassend den Anti-Osteopontin-Antikörper nach einem der Ansprüche 1 bis 2 als aktiven Bestandteil.

4. Verwendung des Anti-Osteopontin-Antikörpers nach einem der Ansprüche 1 bis 2 zur Herstellung eines Medikaments zur Behandlung von Rheumatismus, rheumatoider Arthritis oder Osteoarthritis.

## Revendications

1. Anticorps anti-ostéopontine produit par l'hybridome déposé sous la référence FERM BP-7883.

2. Anticorps anti-ostéopontine qui est produit par humanisation de l'anticorps de la revendication 1.

3. Agent thérapeutique pour traiter le rhumatisme, l'arthrite rhumatoïde ou l'ostéo-arthrite, comprenant en tant qu'ingrédient actif l'anticorps anti-ostéopontine selon l'une quelconque des revendications 1 à 2.

4. Utilisation de l'anticorps anti-ostéopontine selon l'une quelconque des revendications 1 à 2 pour la fabrication d'un médicament pour le traitement du rhumatisme, de l'arthrite rhumatoïde ou de l'ostéo-arthrite.
